(19) Europäisches Patentamt
European Patent Office
Office européen des brevets

(11) Veröffentlichungsnummer: **0 543 343 A2**

# (12) EUROPÄISCHE PATENTANMELDUNG

(21) Anmeldenummer: **92119634.1**

(51) Int. Cl.5: **C07C 227/26**

(22) Anmeldetag: **17.11.92**

(30) Priorität: **19.11.91 CH 3373/91**

(43) Veröffentlichungstag der Anmeldung:
**26.05.93 Patentblatt 93/21**

(84) Benannte Vertragsstaaten:
**AT BE CH DE FR GB IT LI NL SE**

(71) Anmelder: **LONZA AG**

**Gampel/Wallis(CH)**

(72) Erfinder: **Bänziger, Markus, Dr. Chem.**
**Englischgruss– Strasse 42**
**Brig– Glis (Kanton Wallis)(CH)**
Erfinder: **Warm, Aleksander, Dr. Chem.**
**Bifigastrasse**
**Visperterminen (Kanton Wallis)(CH)**
Erfinder: **McGarrity, John, Dr. Chem.**
**Gemsweg 4**
**Visp (Kanton Wallis)(CH)**

(74) Vertreter: **Weinhold, Peter, Dr. et al**
**Patentanwälte Dipl.– Ing. G. Dannenberg Dr. P. Weinhold Dr. D. Gudel Dipl.– Ing. S. Schubert Dr. P. Barz Siegfriedstrasse 8**
**W– 8000 München 40 (DE)**

## (54) Verfahren zur Herstellung von alpha– Hydroxy– beta– aminocarbonsäuren.

(57) α – Hydroxy – β – aminocarbonsäuren (I) werden ausgehend von N – geschützten α – Aminosäurehalogeniden (II) hergestellt.
Die Halogenide (II) werden mit Trimethylsilycyanid in die neuen α – Oxonitrile (Acylcyanide) (III) übergeführt. Diese werden in einer zweiten Stufe in die ebenfalls neuen α – Oxocarbonsäureester (IV) umgewandelt und anschliessend selektiv zu den α – Hydroxycarbonsäureestern (V) reduziert.
Die α – Hydroxycarbonsäureester werden gegebenenfalls in ihre Diastereomeren getrennt und/oder einer Abspaltung der N – Schutzgruppe und/oder einer Esterhydrolyse unterzogen. Die α – Hydroxy – β – aminocarbonsäuren (I) sind Bausteine von Peptiden mit Wirkung als Enzyminhibitor.

EP 0 543 343 A2

Die vorliegende Erfindung betrifft ein Verfahren zur Herstellung von α-Hydroxy-β-aminocarbonsäuren und deren Estern.

Einige α-Hydroxy-β-aminocarbonsäuren sind von Interesse als Bausteine von natürlichen oder synthetischen Peptiden, die Wirksamkeit als Inhibitor bestimmter Enzyme besitzen. Hierzu gehören insbesondere (2R,3S)-3-Amino-2-hydroxy-5-methylhexansäure ("Norstatin") und (2R,3S)-3-Amino-2-hydroxy-4-cyclohexylbuttersäure ("Cyclohexylnorstatin") (s. z.B. K. Iizuka et al., J. Med. Chem. 33 (1990) 2707-2714).

Es sind Verfahren zur Herstellung dieser Verbindungen bekannt, welche ausgehend von den entsprechenden α-Aminocarbonsäuren mit um ein Kohlenstoffatom kürzerer Kette über den N-geschützten α-Aminoalkohol und den entsprechenden N-geschützten α-Aminoaldehyd und das daraus mit Blausäure erhältliche Cyanhydrin durch Hydrolyse der Cyanogruppe zur Carboxylgruppe die gewünschten Produkte liefern (Iizuka et al., loc. cit.; s. auch D. H. Rich et al., J. Org. Chem. 45 (1980) 2288-2290). Ein Nachteil dieser Verfahren ist die Verwendung eines Aldehyds als Zwischenstufe, der entweder durch eine Reduktions-Oxidations-Sequenz aus der Carbonsäure oder durch Reduktion mit Diisobutylaluminiumhydrid aus dem Methylester hergestellt werden muss, was für eine Synthese in grösserem Massstab ungünstig ist. Weitere Probleme ergeben sich aus dem Einsatz von Blausäure.

Ausserdem erfordert die Herstellung von Estern der α-Hydroxy-β-aminosäuren auf diesem Weg einen zusätzlichen Veresterungsschritt.

Aufgabe der vorliegenden Erfindung war daher, ein Verfahren zur Herstellung von α-Hydroxy-β-aminocarbonsäuren und deren Estern bereitzustellen, das die genannten Nachteile vermeidet und auch für die Durchführung im technischen Massstab geeignet ist.

Erfindungsgemäss wird die Aufgabe durch das Verfahren nach Patentanspruch 1 gelöst.

Das erfindungsgemässe Verfahren eignet sich zur Herstellung von α-Hydroxy-β-aminocarbonsäuren und deren Estern der allgemeinen Formel

$$R^1 - \overset{NR^3R^4}{\overset{|}{C^*H}} - \overset{OH}{\overset{|}{C^*H}} - COOR^2 \qquad I$$

worin $R^1$ eine lineare oder verzweigte Alkylgruppe mit 1 bis 6 Kohlenstoffatomen, also beispielsweise Methyl, Ethyl, Propyl, Isopropyl, Butyl, Isobutyl, sec-Butyl, tert-Butyl, Pentyl oder Hexyl ist. Die Alkylgruppe kann mit Arylgruppen, also beispielsweise Phenyl, o-, m- und p-Tolyl, Chlorphenyl, Hydroxyphenyl, Methoxyphenyl, oder Cycloalkylgruppen mit 3 bis 8 Ringgliedern, also beispielsweise Cyclopropyl, Cyclobutyl, Cyclopentyl, Cyclohexyl, Cycloheptyl, Cyclooctyl, substituiert sein. Besonders bevorzugte Gruppen für $R^1$ sind Isopropyl, Isobutyl, sec-Butyl, Benzyl, p-Hydroxybenzyl und Cyclohexylmethyl. Grundsätzlich kann $R^1$ alle Substituenten enthalten, die unter den angewendeten Reaktionbedingungen keine störenden Reaktionen eingehen.

$R^2$ ist Wasserstoff oder eine lineare oder verzweigte Alkylgruppe mit 1 bis 4 Kohlenstoffatomen, also Methyl, Ethyl, Propyl, Isopropyl, Butyl, Isobutyl, sec-Butyl oder tert-Butyl, vorzugsweise Methyl.

$R^3$ und $R^4$ sind unabhängig voneinander Wasserstoff oder Aminoschutzgruppen, oder $R^3$ und $R^4$ bilden zusammen eine bifunktionelle Aminoschutzgruppe.

Unter bifunktionellen Aminoschutzgruppen sind in diesem Zusammenhang solche zu verstehen, die unter Substitution beider Wasserstoffatome einer Aminogruppe zusammen mit dem Amino-Stickstoffatom einen heterocyclischen Ring bilden. Aminoschutzgruppen sind insbesondere in der Peptidsynthese gebräuchlich und dem Fachmann bekannt; eine Übersicht über ihre Eigenschaften und die Methoden zu ihrer Einführung und Abspaltung gibt beispielsweise E. Wünsch, Methoden Org. Chem. (Houben-Weyl) 4th Ed. 1974, Bd. XV/1, S. 46-305.

Die nach dem erfindungsgemässen Verfahren hergestellten Verbindungen besitzen zwei Chiralitätszentren (soweit nicht in $R^1$ und/oder $R^2$ weitere Chiralitätszentren vorhanden sind), können also jeweils in vier stereoisomeren Formen auftreten. Da nur eines der beiden Chiralitätszentren in der erfindungsgemässen Reaktionssequenz neu gebildet wird, während die Konfiguration am anderen Chiralitätszentrum erhalten bleibt, entstehen bei Verwendung optisch aktiver α-Aminosäurederivate als Ausgangsmaterial in der Regel Diastereomerengemische, die aber aufgrund der unterschiedlichen physikalischen Eigenschaften leicht getrennt werden können. Das erfindungsgemässe Verfahren eignet sich deshalb insbesondere zur Herstellung konfigurativ einheitlicher α-Hydroxy-β-aminosäuren.

Als Ausgangsmaterialien werden erfindungsgemäss Chloride oder Bromide, vorzugsweise Chloride von α-Aminocarbonsäuren mit geschützter Aminofunktion entsprechend der allgemeinen Formel

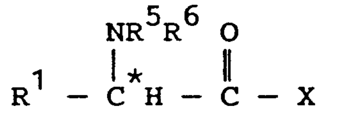

II

eingesetzt. X steht dabei für Chlor oder Brom, $R^1$ hat die gleiche Bedeutung wie in Formel I. $R^5$ und $R^6$ haben die gleiche Bedeutung wie $R^3$ und $R^4$ in Formel I, jedoch mit der Massgabe, dass $R^5$ und $R^6$ nicht beide zugleich Wasserstoff sind. Grundsätzlich kann das Verfahren mit allen Aminoschutzgruppen durchgeführt werden, die nur wenig empfindlich gegen Säure und Basen sowie Trimethylsilylcyanid und -halogenide sind und unter den Reaktionsbedingungen der Stufen a) bis c) gemäss Patentanspruch 1 nicht abgespalten werden. Geeignete Schutzgruppen sind beispielsweise p-Toluolsulfonyl (Tosyl) und von den bifunktionellen Schutzgruppen die sogenannten Diacylschutzgruppen, wie Maleoyl oder insbesondere Phthaloyl. Die Phthaloylschutzgruppe kann nach oder analog zu bekannten Verfahren leicht in α-Aminosäuren eingeführt und mit Hydrazinhydrat oder starken Säuren wieder abgespalten werden (E. Wünsch, loc. cit. S. 250-263).

Die so erhältlichen N-Phthaloyl-α-aminocarbonsäuren können nach ebenfalls bekannten Verfahren mit Halogeniden anorganischer Säuren, wie beispielsweise Thionylchlorid, in die für das erfindungsgemässe Verfahren erforderlichen Aminosäurehalogenide übergeführt werden (s. z.B. P. Stetzel, Methoden Org. Chem. (Houben-Weyl) 4th Ed. 1974 Bd. XV/2, S. 355-364).

Die N-geschützten α-Aminocarbonsäurehalogenide (II) werden erfindungsgemäss in der ersten Stufe a) mit Trimethylsilylcyanid (Cyanotrimethylsilan) in die entsprechenden α-Oxonitrile (Acylcyanide) (III) übergeführt.

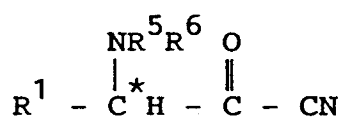

III

Diese Verbindungen sind neu und ebenfalls Gegenstand der vorliegenden Erfindung.

Bei der Reaktion mit Trimethylsilylcyanid findet keine Racemisierung statt, im Gegensatz zu dem bisher bekannten Verfahren der Umsetzung von Carbonsäurehalogeniden mit Kupfer(I)-cyanid, nach dem beispielsweise aus Phthaloylglycylbromid das Phthaloylglycylcyanid hergestellt, bei optisch aktiven α-Aminosäurederivaten aber Racemisierung beobachtet wurde (P. Stetzel, loc. cit. S. 364).

Die Umsetzung mit Trimethylsilylcyanid wird vorzugsweise in Gegenwart einer katalytischen Menge Zinkiodid durchgeführt. Ein Lösungsmittel ist nicht erforderlich, zur besseren Handhabung kann jedoch ein inertes Lösungsmittel, wie beispielsweise Dioxan, Essigsäureethylester oder Tetrahydrofuran zugegeben werden. Die Reaktionstemperatur beträgt vorteilhaft 20 bis 100 ° C, vorzugsweise 40 bis 70 ° C.

Das bei der Reaktion ebenfalls entstehende Trimethylsilylchlorid bzw. -bromid kann aufgrund seines niedrigeren Siedepunkts gegebenenfalls abdestilliert und wieder zur Herstellung von Trimethylsilylcyanid verwendet werden.

Die α-Oxonitrile (III) werden erfindungsgemäss in Stufe b) in die α-Oxocarbonsäureester (IV) übergeführt.

Auch die α-Oxocarbonsäureester (IV) sind neu und ebenfalls Gegenstand der vorliegenden Erfindung.

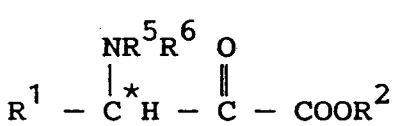

IV

Für die Herstellung der α-Oxocarbonsäureester (IV) ist es nicht notwendig, das α-Oxonitril zu reinigen, sondern es kann das Rohprodukt oder sogar das unaufgearbeitete Reaktionsgemisch der ersten Synthesestufe eingesetzt werden.

Vorzugsweise wird diese Umsetzung nach Art der Pinner-Reaktion mit dem Alkohol, der dem gewünschten Ester entspricht, also vorzugsweise Methanol, und einer starken Säure durchgeführt. Hierbei bildet sich zunächst ein Salz des entsprechenden Iminoesters, das mit Wasser sehr leicht zum normalen Ester hydrolysiert. Die Umsetzung wird vorzugsweise in einem inerten polaren Lösungsmittel, wie beispielsweise Diethylether oder Tetrahydrofuran, durchgeführt. Wegen der als Nebenreaktion möglichen Substitution der Cyanogruppe durch eine Alkoxygruppe, die zu einem α-Aminocarbonsäureester führt, ist es günstig, keinen allzu grossen Alkoholüberschuss zu verwenden. Durch Wahl geeigneter Reaktionsbedingungen kann der Anteil an Substitutionsprodukt auf beispielsweise 5 – 10% beschränkt werden. Als Säure wird vorzugsweise Chlorwasserstoff verwendet, der gasförmig eingeleitet werden kann. Wegen der hohen Reaktivität der α-Oxonitrile kann die Pinner-Reaktion bei verhältnismässig tiefer Temperatur durchgeführt werden.
Die Reaktionstemperatur beträgt zweckmässig −50 bis +20 °C, vorzugsweise −20 bis 0 °C. Es liegt selbstverständlich ebenfalls im Rahmen der Erfindung, die α-Oxocarbonsäureester (IV) nach einer anderen Methode aus den α-Oxonitrilen herzustellen, beispielsweise durch Hydrolyse zur α-Oxocarbonsäure und anschliessende Veresterung.

Die α-Oxocarbonsäureester (IV) werden erfindungsgemäss in Stufe c) selektiv an der Keto-Carbonylgruppe zu den N-geschützten α-Hydroxycarbonsäureestern (V) reduziert. Als Reduktionsmittel werden vorzugsweise Borhydride (Boranate), insbesondere Lithiumborhydrid, Natriumborhydrid, Calciumborhydrid oder Zinkborhydrid, oder Wasserstoff in Gegenwart eines Platin/Rhodium-Mischkatalysators verwendet.
Bei der Reduktion wird ein zweites Asymmetriezentrum gebildet, so dass im allgemeinen zwei Diastereomere bzw. Diastereomerenpaare entstehen, je nachdem, ob von einem optisch aktiven oder von einem racemischen Carbonsäurehalogenid (II) ausgegangen wurde. Das Mengenverhältnis der Diastereomeren, d.h. die Diastereoselektivität der Reduktion, hängt vom verwendeten Reduktionsmittel und der Temperatur ab. Besonders grosse Diastereoselektivitäten wurden mit $LiBH_4$ und $Zn(BH_4)_2$ als Reduktionsmittel beobachtet. Bevorzugt wird jeweils das Diastereomere mit entgegengesetzter Konfiguration an beiden Asymmetriezentren gebildet, also das (2R,3S)- oder das (2S,3R)-Diastereomere.
Die Reduktion mit Borhydriden, vorzugsweise mit $Zn(BH_4)_2$, wird zweckmässig bei einer Temperatur von −50 bis +25 °C durchgeführt, vorzugsweise bei −30 bis 0 °C.
Die Reduktion mit Wasserstoff an einem Platin/Rhodium-Mischkatalysator wird vorzugsweise bei einem Wasserstoffdruck von 10 bis 200 bar und einer Temperatur von 20 bis 80 °C in einem unpolaren Lösungsmittel, wie beispielsweise Toluol, oder einem polaren Lösungsmittel,wie beispielsweise Ethylacetat oder Tetrahydrofuran,durchgeführt.
Es hat sich gezeigt, dass die bei der Reduktion entstehenden Diastereomeren auf dieser Stufe leicht getrennt werden können, beispielsweise durch Säulenchromatographie oder Umkristallisation.
Anschliessend wird gegebenenfalls in einer weiteren Stufe d) die Aminoschutzgruppe abgespalten und/oder die Esterfunktion hydrolysiert. Beispielsweise kann die Phthaloyl-Schutzgruppe unter Erhalt der Esterfunktion mit Hydrazinhydrat oder unter Hydrolyse des Esters mit einer starken Säure abgespalten werden. Unter weniger drastischen Bedingungen ist es dagegen möglich, mit Säure nur die Estergruppe unter Erhalt der Phthaloyl-Schutzgruppe zu hydrolysieren.

Die nachfolgenden Beispiele verdeutlichen die Durchführung des erfindungsgemässen Verfahrens.

## Beispiel 1

### N-Phthaloyl-L-phenylalanylchlorid

((S)-3-Phenyl-2-phthalimidopropionylchlorid) Eine Mischung von 138,2 g (0,47 mol) N-Phthaloyl-L-phenylalanin (hergestellt nach A. K. Bose, Org. Syntheses, Coll. Vol. 5, S. 973) und 167 g (1,4 mol) Thionylchlorid wurde 3 h zum Rückfluss erhitzt. Anschliessend wurde das überschüssige Thionylchlorid abdestilliert. Der Rückstand wurde mit 200 ml Toluol versetzt und auf ca. 80 °C erhitzt. Nach Zugabe von 380 ml Hexan wurde die Lösung zur Kristallisation auf ca. 4 °C abgekühlt. Das auskristallisierte Produkt wurde abfiltriert und getrocknet.

| | |
|---|---|
| Ausbeute: | 143,9 g (98% d.Th.) |
| Schmp.: | 81,9 – 82,4 °C |
| $[\alpha]_D^{25}$ : | −226,7° (c = 2,0; Benzol) |
| $^1H$-NMR ($CDCl_3$, 300 MHz) δ: | 3,50 – 3,72 (m, 2H); |
| | 5,35 (dd, 1H); |
| | 7,10 – 7,24 (m, 5H); |
| | 7,65 – 7,79 (m, 4H). |

Beispiel 2

(S)-2-Oxo-4-phenyl-3-phthalimidobutannitril

Eine Mischung aus 25 g (79,7 mmol) N-Phthaloyl-L-phenylalanylchlorid (hergestellt nach Beispiel 1), 9,5 g (95,7 mmol) Trimethylsilylcyanid und 255 mg (0,80 mmol) Zinkiodid wurde auf 60 °C erhitzt. Nach 35 min wurden 30 ml Tetrahydrofuran zugegeben. Das Gemisch wurde noch 80 min am Rückfluss gekocht, anschliessend mit 200 ml Hexan versetzt und auf ca. 4 °C abgekühlt. Das ausgefallene Produkt wurde abfiltriert, mit Hexan gewaschen und im Vakuum getrocknet.

| | |
|---|---|
| Ausbeute: | 21,06 g (87% d.Th) |
| $[\alpha]_D^{20}$ : | −264,2° (c = 1,0; THF) |
| $^1H$-NMR ($CDCl_3$, 300 MHz) δ: | 3,38 (dd, 1H); |
| | 3,65 (dd, 1H); |
| | 5,20 (dd, 1H); |
| | 7,08−7,23 (m, 5H); |
| | 7,70−7,90 (m, 4H). |

Beispiel 3

(S)-2-Oxo-4-phenyl-3-phthalimidobuttersäuremethylester

Ein Gemisch aus 100 ml Diethylether und 4 ml Methanol wurde bei −10 °C mit HCl-Gas gesättigt. Anschliessend wurde 9,0 g (30 mmol) (S)-2-Oxo-4-phenyl-3-phthalimidobutannitril (hergestellt nach Beispiel 2) zugegeben. Das Gemisch wurde bei −10 °C 45 min gerührt und anschliessend bei −20 bis 0 °C mit 170 ml Wasser versetzt.

Die wässrige Phase wurde dreimal mit je 200 ml Diethylether extrahiert. Die vereinigten Etherphasen wurden dreimal mit je 150 ml gesättigter Natriumhydrogencarbonatlösung gewaschen und die Waschlösung mit Diethylether zurückextrahiert. Die vereinigten Etherphasen wurden dann über Magnesiumsulfat getrocknet, am Rotationsverdampfer eingeengt und schliesslich im Hochvakuum vom restlichen Lösungsmittel befreit.

| | |
|---|---|
| Ausbeute: | 6,8 g (68% d.Th.) dickflüssiges Öl |
| $^1H$-NMR ($CDCl_3$, 300 MHz) δ: | 3,40 (dd, 1H); |
| | 3,60 (dd, 1H); |
| | 3,84 (s, 3H); |
| | 5,57 (dd, 1H); |
| | 7,10−7,25 (m, 5H); |
| | 7,65−7,87 (m, 4H). |

Nach chromatographischer Reinigung konnte das Produkt kristallisiert werden:

| | |
|---|---|
| Schmp.: | 75−76 °C |
| $[\alpha]_D^{20}$ : | −200,4° (c = 1,0; $CHCl_3$) |

Beispiel 4

(2R,3S)- und (2S,3S)-2-Hydroxy-4-phenyl-3-phthalimidobuttersäuremethylester

In 50 ml Tetrahydrofuran wurden 4,92 g (S)-2-Oxo-4-phenyl-3-phthalimidobuttersäuremethylester (hergestellt nach Beispiel 3) gelöst. Die Lösung wurde auf −25 °C abgekühlt und mit 95 mg Lithiumborhydrid versetzt. Das Reaktionsgemisch wurde 30 min bei −25 °C bis −20 °C gerührt. Danach wurden 200 ml Diethylether zugegeben und das Gemisch zweimal mit je 100 ml 0,1 M Salzsäure gewaschen. Die Waschlösungen wurde zweimal mit je 100 ml Diethylether zurückextrahiert. Die vereinigten organischen Phasen wurden über Magnesiumsulfat getrocknet, am Rotationsverdampfer eingeengt und schliesslich im Hochvakuum von Lösungsmittelresten befreit.

Ausbeute: 4,78 g (96% d.Th.) (Öl)

Durch Integration der $^1H$-NMR-Signale wurde das Diastereomerenverhältnis (2R,3S):(2S,3S) zu ca. 5:1 bestimmt.

(Die Konfigurationszuordnung erfolgte nach Trennung, Abspaltung der Phthaloylgruppe und Derivatisierung mit Phosgen zu dem entsprechenden Oxazolidinon aufgrund der Kopplungskonstanten im $^1H$-NMR, vgl. dazu K. Iizuka et al., J. Med. Chem. **1990**, 33, 2707−2714).

Das Diastereomerengemisch konnte durch Chromatographie an Kieselgel (Laufmittel Hexan/Dichlormethan/Diethylether 2:2:1) gereinigt und getrennt werden.

(2R,3S)-Verbindung:

| | |
|---|---|
| Schmp.: | 91,9-92,9 °C |
| $[\alpha]_D^{24}$ : | -107,6° (c = 1,0; $CHCl_3$) |
| $^1H$-NMR ($CDCl_3$, 300 MHz) δ: | 3,20-3,40 (m, 2H); |
| | 3,63 (s, 3H); |
| | 4,48-4,60 (m, 2H); |
| | 4,90-5,00 (m, 1H); |
| | 7,10-7,30 (m, 5H); |
| | 7,67-7,85 (m, 4H). |

Beispiel 5

(2R,3S)-3-Amino-2-hydroxy-4-phenylbuttersäure-hydrochlorid

In 60 ml Essigsäure wurden 13,25 g (39,1 mmol) (2R,3S)-/(2R,3S)-2-Hydroxy-4-phenyl-3-phthalimidobuttersäuremethylester (Diastereomerenverhältnis = 3:1) gelöst. Die Lösung wurde mit 380 ml konzentrierter Salzsäure versetzt und 8 h zum Rückfluss erhitzt. Nach dem Abkühlen wurde die Mischung 18 h kontinuierlich in einem Flüssig-flüssig-Extraktor (nach Kutscher-Steudel) mit Diethylether extrahiert. Der Etherextrakt und die wässrige Phase wurden separat eingedampft. Der Rückstand der Etherphase (7,41 g braunes Pulver) bestand im wesentlichen aus Phthalsäure. Der Rückstand der Wasserphase (6,38 g) wurde in 200 ml Acetonitril aufgeschlämmt und ca. 15 min zum Rückfluss erhitzt. Der ungelöste Teil (2,6 g) wurde abfiltriert, er bestand im wesentlichen aus dem (2R,3S)-Diastereomeren.

| | |
|---|---|
| $^1H$-NMR (DMSO-$d_6$, 300 MHz): δ: | 2,90 (dd, 1H); |
| | 3,05 (dd, 1H); |
| | 3,40 (br.m, 1H); |
| | 3,90 (d, 1H); |
| | 7,20-7,43 (m, 5H); |
| | 8,20 (br.m, 3H). |

Beispiel 6

(2R,3S)-3-Amino-4-cyclohexyl-2-hydroxybuttersäure-hydrochlorid (Cyclohexylnorstatin-hydrochlorid)

In 5 ml Essigsäure und 5 ml Wasser wurden 500 mg (2R,3S)-3-Amino-2-hydroxy-4-phenylbuttersäurehydrochlorid gelöst, mit 50 mg Rhodium/Aktivkohle (5% Rh) versetzt und bei 20 bar Wasserstoffdruck 24 h bei Raumtemperatur hydriert. Anschliessend wurde der Katalysator abfiltriert und das Filtrat am Rotationsverdampfer eingeengt. Der Rückstand wurde im Hochvakuum getrocknet.

| | |
|---|---|
| Ausbeute: | quantitativ |
| $^1H$-NMR (DMSO-$d_6$, 300 MHz): δ: | 0,75-1,80 (m, 13H); |
| | 3,40 (br.m, 1H); |
| | 4,08 (d, 1H). |

Beispiel 7

(2R,3S)-2-Hydroxy-4-phenyl-3-phthalimidobuttersäure

In 50 ml Essigsäure wurden 16,8 g (2R,3S)-2-Hydroxy-4-phenyl-3-phthalimidobuttersäuremethylester (Gehalt nach HPLC 49,6%) gelöst und mit 100 ml 32%-iger Salzsäure versetzt. Das Gemisch wurde 28 h bei Raumtemperatur gerührt, wobei ein weisser Feststoff ausfiel. Der Niederschlag wurde abfiltriert und mit kaltem Wasser gewaschen.

| | |
|---|---|
| Ausbeute: | 10,2 g |
| $^1H$-NMR ($CDCl_3$, 300 MHz): δ: | 3,15-3,40 (m, 2H); |
| | 4,50 (d, 1H); |

4,95 – 5,05 (m, 1H);
7,08 – 7,20 (m, 5H);
7,60 – 7,80 (m, 4H).

Beispiel 8

(2R,3S) – 2 – Hydroxy – 4 – phenyl – 3 – phthalimidobuttersäuremethylester (Reduktion mit $Zn(BH_4)_2$)

In 585 ml wasserfreiem Tetrahydrofuran wurden 2,85 g Natriumborhydrid und 12,83 g wasserfreies Zinkchlorid 18 h bei Raumtemperatur gerührt. Anschliessend wurde die entstandene Suspension auf – 25 ° C abgekühlt und mit einer Lösung von 63,5 g (S) – 2 – Oxo – 4 – phenyl – 3 – phthalimidobuttersäure – methylester in 50 ml Tetrahydrofuran versetzt.
Das Gemisch wurde noch 1,5 h bei – 25 ° C gerührt, anschliessend mit 500 ml Toluol verdünnt und dreimal mit je 350 ml 0,1 M Salzsäure extrahiert. Die organische Phase wurde dreimal mit je 350 ml gesättigter Natriumhydrogencarbonatlösung gewaschen. Die vereinigten wässrigen Phasen wurden mit Toluol extra – hiert. Nach Trocknung der vereinigten organischen Phasen über Magnesiumsulfat wurde das Lösungsmittel am Rotationsverdampfer abdestilliert und der Rückstand aus 160 ml Toluol umkristallisiert.
Ausbeute: 33,9 g (53% d.Th)

Beispiel 9

(2R,3S) – 2 – Hydroxy – 4 – phenyl – 3 – phthalimidobuttersäuremethylester (Reduktion mit $Ca(BH_4)_2$)

Im 210 ml wasserfreien Tetrahydrofuran wurden 1,12 g Natriumborhydrid und 1,23 g wasserfreies Calciumchlorid 16 h bei Raumtemperatur gerührt. Anschliessend wurde das Gemisch auf – 25 ° C abge – kühlt. Zu diesem Gemisch wurde eine Lösung von 25 g (S) – 2 – Oxo – 4 – phenyl – 3 – phthalimidobutter – säuremethylester in 40 ml Tetrahydrofuran zugetropft. Das Reaktionsgemisch wurde noch 30 min bei – 25 ° C gerührt, dann mit 250 ml Toluol verdünnt und erst mit 200 ml 0,1 M Salzsäure und schliesslich dreimal mit je 250 ml gesättigter Natriumhydrogencarbonatlösung gewaschen. Die weitere Aufarbeitung erfolgte wie in Beispiel 8 beschrieben, jedoch ohne Umkristallisation.
Ausbeute: 22,5 g (89,5%, d.Th)
Diastereomerenverhältnis: (2R,3S)/(2S,3S) ≃ 4:1

Beispiel 10

(2R,3S) – 2 – Hydroxy – 4 – phenyl – 3 – phthalimidobuttersäuremethylester (Reduktion mit $H_2$/Pt/Rh)

In einer Lösung von 1 g (S) – 2 – Oxo – 4 – phenyl – 3 – phtalimidobuttersäuremethylester in 10 ml Ethylacetat wurden 100 mg eines Pt/Rh – Mischkatalysators (4% Pt, 1% Rh auf Aktivkohle) suspendiert. Die Mischung wurde bei 50 bar Wasserstoffdruck und 50 ° C 24 h hydriert, anschliessend vom Katalysator abfiltriert und am Rotationsverdampfer eingeengt.
Ausbeute: 1,0 g, Gehalt (HPLC) 53,5%
Diastereomerenverhältnis: (2R, 3S) ≃ 5,7:1

## Patentansprüche

**1.** Verfahren zur Herstellung von α – Hydroxy – β – aminocarbonsäuren und deren Estern und Salzen der allgemeinen Formel

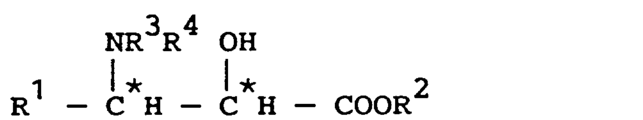

I

worin $R^1$ eine lineare oder verzweigte Alkylgruppe mit 1 bis 6 Kohlenstoffatomen, die gegebenenfalls mit einer Arylgruppe oder einer Cycloalkylgruppe mit 3 bis 8 Ringgliedern substituiert ist, $R^2$ Wasser – stoff oder eine lineare oder verzweigte Alkylgruppe mit 1 bis 4 Kohlenstoffatomen ist und $R^3$ und $R^4$

unabhängig voneinander für Wasserstoff oder eine Aminoschutzgruppe oder $R^3$ und $R^4$ zusammen für eine bifunktionelle Aminoschutzgruppe stehen, dadurch gekennzeichnet, dass ein N-geschütztes α-Aminocarbonsäurehalogenid der allgemeinen Formel

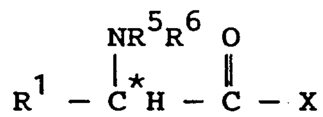

II

worin $R^1$ die oben genannte Bedeutung hat, X Chlor oder Brom ist und $R^5$ und $R^6$ die oben für $R^3$ und $R^4$ genannte Bedeutung haben, mit der Massgabe, dass $R^5$ und $R^6$ nicht beide zugleich Wasserstoff sind,

a) in einer ersten Stufe mit Trimethylsilylcyanid zum entsprechenden α-Oxonitril der allgemeinen Formel

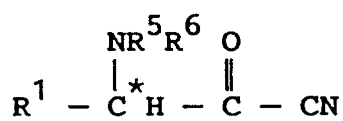

III

worin $R^1$, $R^5$ und $R^6$ die genannten Bedeutungen haben, umgesetzt wird, welches

b) durch Umwandlung der Cyanogruppe in eine Alkoxycarbonylgruppe in den entsprechenden α-Oxocarbonsäureester der allgemeinen Formel

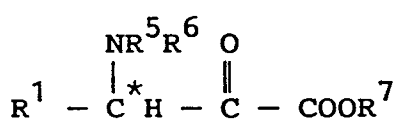

IV

worin $R^1$, $R^5$ und $R^6$ die oben genannten Bedeutungen haben, und $R^7$ die für $R^2$ genannte Bedeutung hat, mit der Massgabe, dass $R^7$ nicht Wasserstoff ist, übergeführt,

c) durch selektive Reduktion der Ketogruppe in den entsprechenden N-geschützten α-Hydroxycarbonsäureester der allgemeinen Formel

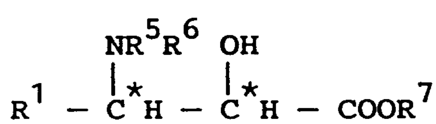

V

worin $R^1$ und $R^5$ bis $R^7$ die oben genannten Bedeutungen haben, umgewandelt und gegebenenfalls

d) die Aminoschutzgruppe abgespalten und/oder die Esterfunktion hydrolysiert wird.

**2.** Verfahren nach dem Patentanspruch 1, dadurch gekennzeichnet, dass das N-geschützte α-Aminocarbonsäurehalogenid (II) in der L-Konfiguration eingesetzt wird.

**3.** Verfahren nach Patentanspruch 1 oder 2, dadurch gekennzeichnet, dass als N-geschütztes α-Aminocarbonsäurehalogenid (II) ein N-geschütztes α-Aminocarbonsäurechlorid, z.B. N-geschütztes L-Phenylalanylchlorid eingesetzt wird.

**4.** Verfahren nach einem oder mehreren der Patentansprüche 1 bis 3, dadurch gekennzeichnet, dass als Aminoschutzgruppe $R^5R^6$ die Phthaloylgruppe eingesetzt wird.

**5.** Verfahren nach Patentanspruch 4, dadurch gekennzeichnet, dass die Abspaltung der Phthaloylgruppe mit Hydrazinhydrat oder konzentrierter Salzsäure durchgeführt wird.

**6.** Verfahren nach einem oder mehreren der Patentansprüche 1 bis 5, dadurch gekennzeichnet, dass die Umsetzung mit Trimethylsilylcyanid in Gegenwart von Zinkiodid durchgeführt wird.

**7.** Verfahren nach einem oder mehreren der Patentansprüche 1 bis 6, dadurch gekennzeichnet, dass die Umwandlung der Cyanogruppe in eine Alkoxycarbonylgruppe durch Umsetzung mit dem entsprechenden Alkohol $R^7OH$ in Gegenwart einer starken Säure und anschliessende Hydrolyse des als Zwischenprodukt gebildeten Iminoester-Salzes vorgenommen wird.

**8.** Verfahren nach Patentanspruch 7, dadurch gekennzeichnet, dass als Alkohol Methanol eingesetzt wird.

**9.** Verfahren nach einem oder mehreren der Patentansprüche 1 bis 8, dadurch gekennzeichnet, dass zwischen Schritt c) und Schritt d) eine Diastereomerentrennung der $\alpha$-Hydroxycarbonsäureester (V) durchgeführt wird.

**10.** Verfahren nach einem oder mehreren der Patentansprüche 1 bis 9, dadurch gekennzeichnet, dass die selektive Reduktion der Ketogruppe mit einem Borhydrid aus der Gruppe $LiBH_4$, $NaBH_4$, $Ca(BH_4)_2$, $Zn(BH_4)_2$ als Reduktionsmittel oder durch katalytische Hydrierung an einem Platin/Rhodium-Mischkatalysator durchgeführt wird.

**11.** N-geschützte $\alpha$-Oxo-$\beta$-aminonitrile der allgemeinen Formel

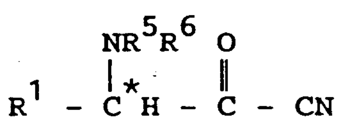

III

worin $R^1$ eine lineare oder verzweigte Alkylgruppe mit 1 bis 6 Kohlenstoffatomen, die gegebenenfalls mit einer Arylgruppe oder einer Cycloalkylgruppe mit 3 bis 8 Ringgliedern substituiert ist, und entweder $R^5$ eine Aminoschutzgruppe und $R^6$ Wasserstoff oder eine Aminoschutzgruppe ist oder $R^5$ und $R^6$ zusammen eine bifunktionelle Aminoschutzgruppe bilden.

**12.** Verbindungen nach Patentanspruch 11, dadurch gekennzeichnet, dass sie (S)-Konfiguration besitzen.

**13.** N-geschützte $\alpha$-Oxo-$\beta$-aminocarbonsäureester der allgemeinen Formel

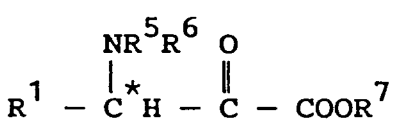

IV

worin das Asymmetriezentrum die (S)-Konfiguration besetzt, $R^1$ eine lineare oder verzweigte Alkylgruppe mit 1 bis 6 Kohlenstoffatomen, die gegebenenfalls mit einer Arylgruppe oder einer Cycloalkylgruppe mit 3 bis 8 Ringgliedern substituiert ist, und $R^7$ eine lineare oder verzweigte Alkylgruppe mit 1 bis 4 Kohlenstoffatomen ist und entweder $R^5$ eine Aminoschutzgruppe und $R^6$ Wasserstoff oder eine Aminoschutzgruppe ist oder $R^5$ und $R^6$ zusammen eine bifunktionelle Aminoschutzgruppe bilden.

**14.** Verbindungen nach Patentanspruch 13, dadurch gekennzeichnet, dass $R^7$ Methyl ist.

**15.** Verbindungen nach einem oder mehreren der Patentansprüche 11 bis 14, dadurch gekennzeichnet, dass $R^5$ und $R^6$ zusammen eine Phthaloylgruppe sind.

**16.** Verbindungen nach einem oder mehreren der Patentansprüche 11 bis 15, dadurch gekennzeichnet, dass $R^1$ ausgewählt ist aus der Gruppe bestehend aus Isobutyl, Benzyl, p-Hydroxybenzyl und

Cyclohexylmethyl.